Europäisches Patentamt

European Patent Office

Office européen des brevets

(11) Publication number: **0 324 254 B1**

# EUROPEAN PATENT SPECIFICATION

(45) Date of publication of patent specification: **09.09.92**

(51) Int. Cl.⁵: **A01N 63/00**, C12N 1/20, C12N 15/00, //(C12P1/04, C12R1:07)

(21) Application number: **88312060.2**

(22) Date of filing: **20.12.88**

(54) Bacillus Thuringiensis strain.

(30) Priority: **13.01.88 GB 8800652**

(43) Date of publication of application:
**19.07.89 Bulletin 89/29**

(45) Publication of the grant of the patent:
**09.09.92 Bulletin 92/37**

(84) Designated Contracting States:
**AT BE CH DE ES FR GB GR IT LI LU NL SE**

(56) References cited:
**EP-A- 0 142 924**
**EP-A- 0 149 162**
**EP-A- 0 202 739**
**EP-A- 0 228 228**
**EP-A- 0 238 311**

**BIOLOGICAL ABSTRACTS, no. 87046633; W.P. DONOVAN et al.: "Isolation and characterization of EG2158 a new strain of bacillus-thuringiensis toxic to coleopteran larvae and nucleotide sequence of the toxin gene", & MOLECULAR & GENERAL GENETICS(WEST GERMANY) 1988, vol. 214, no. 3, pages 365-372**

(73) Proprietor: **IMPERIAL CHEMICAL INDUSTRIES PLC**
**Imperial Chemical House, Millbank**
**London SW1P 3JF(GB)**

(72) Inventor: **Bernier, Roger Laurent**
**465 Woodview Road, Unit 3**
**Burlington Ontario, L7N 2Z9(CA)**
Inventor: **Collins, Martyn Davies**
**27 Barlavington Way**
**Midhurst West Sussex, GU29 9TG(CA)**
Inventor: **Gray, Ann Louise**
**2173 Galena Crescent**
**Oakville Ontario, L6H 4B1(CA)**

(74) Representative: **Roberts, Timothy Wace et al**
**ICI Group Patents Services Dept. PO Box 6**
**Shire Park Bessemer Road**
**Welwyn Garden City, Herts, AL7 1HD(GB)**

BIOLOGICAL ABSTRACTS, no. 85043878; A. KRIEG et al.: "Bacillus-thuringiensis-var-San-Diego strain M-7 is identical to the formerly in Germany isolated strain BI 256-82 bacillus-thuringiensis-SSP-tenebrionis which is pathogenic to coleopteran insects", & J. APPL. ENTOMOL. 1987, vol. 104, no. 4, pages 417-424

**Description**

The present invention relates to a novel bacterial strain, and in particular to a novel strain of the bacterium Bacillus thuringiensis and uses therefor.

The organism Bacillus thuringiensis produces a protein crystal endotoxin which kills insects. It is not however toxic to mammals. It is thus very useful as an agricultural insecticide, in particular against Lepidoptera, and strains of Bacillus thuringiensis have been used as agricultural insecticides for a number of years.

The strain of Bacillus thuringiensis which is most commonly used commercially is HD-1 (available from the collection of Bacillus thuringiensis strains maintained by the US Department of Agriculture). We have now discovered a novel strain of Bacillus thuringiensis having generally similar properties to HD-1, but distinguished therefrom by improved insecticidal activity against coleopteran pests.

According to the present invention we provide the novel strain A30 of Bacillus thuringiensis, deposited at National Collection of Industrial and Marine Bacteria under the accession number NCIB 12572. We further provide novel insecticidal compositions characterised in that they contain the $\delta$-endotoxin produced by said strain A30, and a method of protecting plants from insect attack which comprises exposing the insects to the $\delta$-endotoxin produced by the said strain A30.

The strain A30 was isolated from chopped grain from Dalrymple, Ontario, Canada. In colony morphology it is generally similar to HD-1. The morphology of the two strains is compared in Table 1.

TABLE 1 – MORPHOLOGY

| Strain | Crystals | Cell Morphology | Colony Morphology |
|---|---|---|---|
| HD-1 | Medium bipyramids, plus undefined shaped crystals | Rods in pairs with terminal spores which do not distend the cell | Lecithinase negative, round colonies; umbonate, yellow centres; 1.0 cm diameter |
| A30 | Large and small bipyramids; triangles and irregular crystals | Single rods or short chains; terminal non-distending spores | Lecithinase positive; teardrop shape colonies; umbonate; yellow centre; 1.0 cm x 0.7 cm |

Biochemical properties of the two strains are compared in Tables 2-4.

4

TABLE 2

| Biochemical Markers on Microtitre Plate | | |
|---|---|---|
| Reagent | HD-1 | A30 |
| MR | + | ( + ) |
| VP | + | + |
| Starch | + | + |
| DNAse | - | ( + ) |
| Urease | + | Y |
| Mannose | - | - |
| Sucrose | - | - |
| Cellobiose | - | - |
| Glucose | + | + |
| Maltose | + | - |
| Salicin | + | - |
| Thorneleys | + | - |
| - : Negative Reaction; + : Positive Reaction; ( + ) : Partial Reaction Y : Yellow colour develops | | |

TABLE 3

| Biochemical Markers on ID-IDENT Plates | | |
|---|---|---|
| | HD-1 | A30 |
| Indole production | - | - |
| N-acetyl-glucosaminidase | - | - |
| $\alpha$-glucosidase | - | - |
| $\alpha$-arabinosidase | - | - |
| $\beta$-glucosidase | - | - |
| $\alpha$-fucosidase | - | - |
| phosphatase | - | - |
| $\alpha$-galactosidase | - | - |
| $\beta$-galactosidase | - | - |
| Inoxyl acetate | + | + |
| Use of arginine | - | - |
| Leucine aminopeptidase | - | - |
| Proline aminopeptidase | - | - |
| Pyroglutamic acid arylamidase | - | - |
| Tyrosine aminopeptidase | - | - |
| Arginine aminopeptidase | + | + |
| Alanine aminopeptidase | - | - |
| Histidine aminopeptidase | - | - |
| Phenylalanine aminopeptidase | - | + |
| Glycine | - | - |
| Catalase | + | + |
| ID-IDENT is a Trade Mark of API Analytab Products | | |

TABLE 4

| Biochemical Markers on API-ZYME Plates | | |
|---|---|---|
| | HD-1 | A-30 |
| Control | 0 | 0 |
| Alkaline phosphatase | 0 | 0 |
| Esterase (C-4) | 0 | 0 |
| Esterase Lipase(C-8) | 0 | + |
| Esterase Lipase(C-14) | 0 | 0 |
| Leucine aminopeptidase | + | + |
| Valine aminopeptidase | 0 | 0 |
| Cysteine aminopeptidase | 0 | 0 |
| Trypsin aminopeptidase | 0 | 0 |
| Chymotrypsin | 0 | 0 |
| Acid phosphatase | 0 | 0 |
| Phosphoamidase | 0 | 0 |
| $\alpha$-galactosidase | 0 | 0 |
| $\beta$-galactosidase | 0 | 0 |
| $\beta$-glucuronidase | 0 | 0 |
| $\alpha$-glucosidase | 0 | 0 |
| $\beta$-glucosidase | 0 | 0 |
| $\eta$-acetyl-$\beta$-glucosaminidase | 0 | 0 |
| $\alpha$-mannosidase | 0 | 0 |
| $\alpha$-fucosidase | 0 | 0 |
| "API-ZYME" is a Trade Mark of API Analytab Products | | |
| O = No reaction + = Positive reaction | | |

In view of these similarities, it is surprising that A30 shows significantly improved insecticidal activity over coleopteran pests as compared with HD-1.

The strain according to the invention may be prepared in any quantity required by fermenting a sample of NCIB 12572 obtained from the National Collections of Industrial and Marine Bacteria under suitable conditions in an appropriate medium. Such conditions and media are well known to the art. The media will, for example, generally contain a nitrogen source (eg fish protein) and a carbohydrate source such as starch. Suitable conditions include a temperature in the range 15-45°C, and an approximately neutral pH. Fermentation may be conveniently carried out in batches, typically for periods of 1-3 days.

Insecticidal compositions according to the invention may be obtained from the fermentation liquor by concentration,for example by centrifugation or filtration followed by addition of any desired and appropriate formulating agents. Formulating agents which may be useful include for example surface active agents, eg, wetting agents: solid diluents, dispersing agents and UV stabilisers. If desired, solid formulations may be prepared by known methods.

The process of the invention is generally carried out by treating (eg spraying) plants infested or liable to infestation by insects with insecticidal compositions as described above diluted with a diluent such as water. The insecticidal agent is the toxic δ-endotoxin: if desired this may be applied to the plants or insects infesting them independently of the bacteria that produce it. Separation of the crystalliferous protein from the bacteria is generally not necessary.

One method of carrying out the process of the invention is to arrange for the plant susceptible to insect attack to produce the δ-endotoxin in situ. This is done by cloning a δ-endotoxin gene from the strain NCIMB 12572, by known means; providing it with a suitable promoter (for example the CaMV35S promoter) which will cause expression of the gene in plants, and transforming the plant by known methods (eg, the use of Ti plasmids). Such processes are described in more detail in EPA 142924 (Agrigenetics), the disclosure of which is incorporated herein by reference.

Insects which are combated by the process of the invention include coleoptera, for example, those in Table 5 below.

TABLE 5

| Common Name | Latin Name |
|---|---|
| Colorado potato beetle | Leptinotarsa decemlineata |
| Corn root worm | Diabrotica undecimpunctata howardi |
| Boll weevil | Anthonomus grandis |

The process of the invention may be used to protect a wide variety of plants prone to infestation by coleoptera. Specific examples of commercially important plants to be protected by the invention are maize (corn) and conifers: as well as cotton, potatoes, rice, small grain cereals such as wheat and barley, and vegetables including lettuce, tomatoes and sugar beet.

The following Examples illustrate the invention.

EXAMPLE 1

Isolation of the Bt strain A30 according to the invention.

Chopped grain sample isolated in Dalrymple, Ontario was diluted by placing 5.0g of the sample into a dilution bottle containing 45 ml of 0.05% peptone to give a $10^{-1}$ dilution. The sample was then heat treated by transferring it to a 60°C water bath for 10 minutes. Sequential dilutions were made by taking 0.5 ml of the sample with the highest dilution and placing it into 4.5 ml of 0.05% peptone (ie. 0.5 ml of the $10^{-1}$ dilution into 4.5 ml peptone diluent yields as to $10^{-2}$ dilution); this was repeated until a $10^{-5}$ dilution was obtained. B. cereus selective medium (Bacillus cereus agar base, Code CM617 from Oxoid, Canada) and esculin agar (in g/L of water: esculin 1.0; ferric citrate 0.5; peptone 10; NaCl 5; Agar 20) were used to plate $10^{-3}$ to $10^{-6}$ dilutions. The plated samples were incubated at 30°C for 5 days and then examined for potential Bt colonies. Slides were made of the chosen colonies using the Smirnoff staining procedure and were examined under the microscope (at a magnification of 1000X using the oil immersion lens) for the presence of darkly staining parasporal crystals which were usually but not necessarily bipyramidal in shape.

Crystal-positive colonies were streaked onto nutrient agar in order to ensure a pure culture, and incubated for another 5 days at 30°C. The Smirnoff staining procedure was repeated to confirm crystal presence, and to check purity. Purified colonies were transferred to nutrient agar slants and stored in a refrigerator at 4°C for further use.

EXAMPLE 2

Propagation of the Bt strain A30 according to the invention.

Inoculum was transferred from a slant to a 250 ml Erlenmeyer flask containing 100 ml of CRL#1 medium (in g or ml/liter of water: nutrient broth 8; glucose 6; yeast extract 5; xylose 0.5; cotton seed flour extract 30 ml; corn steep liquor 3.2 ml; Mary Mendel's salt mixture 1 ml) and incubated wtih agitation at 30°C and 3400 rpm. After 24 hours, the entire 100 ml was used to inoculate 1 liter of the same medium in a 2L flask; this was incubated with agitation for 5 days at 300 rpm at 30°C. The cells, spores and crystals were then harvested by centrifugation and acetone-precipitated using the Dulmage method.

EXAMPLE 3

Formulation according to the invention.

Upon completion of the fermentation cycle, A30 bacteria were harvested by first separating the Bt spores and crystals from the fermentation broth as described in Example 2. The recovered spores and crystals were resuspended in 100 ml of water and formulated into a liquid concentrate by adding 4.9g of *Morwet D-425 (dispersing agent), 4.9g of *Veegum HV (suspending agent), 4.9 ml of *Tween 80 (wetting agent) and 24.4 ml of *Sorbo (anti-freezing agent). Each ingredient was added separately in order stated above. The product was kept at 4°C prior to use. A similar formulation was performed using HD-1, to serve as a control.

Examples 4-7 illustrate the activity of the novel Bt strain against different coleopteran insects.

*Registreed    Trademarks

EXAMPLE 4

Potato leaves were dipped in a suspension of A30 spores and crystals (obtained as described in Example 2) at various concentrations and used to feed 1 day-old Colorado potato beetle larvae. Results are shown in Table 6. HD-1 had no effect at these concentrations.

TABLE 6

| Rate ($\mu$g powder/ml of solution) | % mortality (4 DAT) | % feeding reduction* (4 DAT) |
|---|---|---|
| 6400 | 81 | 90 |
| 3200 | 81 | 86 |
| 1600 | 77 | 87 |
| 800 | 48 | 69 |
| 400 | 23 | 30 |

*Measure of reduction in % leaf area consumed as compared with % leaf area consumed in untreated control.

EXAMPLE 5

A mixture of Bt spores and crystals was prepared by incubating strain A30 at 30°C for five days on 210mm x 210mm Petri plates containing L nutrient agar (10g tryptone, 10g yeast extract, 5g NaCl, 10g agar per litre), scraping confluent growth from the agar surface and freeze-drying. Freeze-dried sporesand crystals were mixed with a sterile 8% sucrose solution to a final concentration of 4800 ppm. This solution was used as the food source for Western Corn Rootworm (Diabrotica virgifera virgifera), using 25 first-instar larvae per test. Results are shown in Table 7.

TABLE 7

| Replicate | % Mortality at 3 DAT | |
|---|---|---|
| | Strain A30 | Untreated control |
| 1 | 13 | 2 |
| 2 | 10 | 2 |
| (DAT = Days After Treatment) | | |

EXAMPLE 6

A mixture of A30 spores and crystals was prepared as in Example 5. Freeze-dried spores and crystals were mixed with a sterile 2.5% sucrose solution to a final concentration of 2000ppm. This solution was used as the food source for Southern Corn Rootworm (Diabrotica undecimpunctata howardi). A similar formulation containing the lepidopteran-active strain A20 (deposited with the National Collections of Industrial and Marine Bacteria under the number NCIB 12570) was used as a control. Results are shown in Table 8.

TABLE 8

| Bt strain | % Mortality at 3 DAT |
|---|---|
| A30 | 26 |
| A20 (control) | 0 |

EXAMPLE 7

A mixture of A30 spores and crystals was prepared as in Example 5. The freeze-dried mixture was used to dip cotton cotyledons which were then infested with either larvae or adults of the boll weevil (Anthonomus grandis). Results, recorded after three days, are shown in Table 9.

TABLE 9

| Insect stage | % Mortality | % Feeding reduction |
|---|---|---|
| Adults | 22 | 74 |
| Larvae | 25 | 43 |

The mortality of untreated (control) larvae was 2%

**Claims**

1. The novel strain A30 of Bacillus thuringiensis, deposited at the National Collections of Industrial and Marine Bacteria Scotland under the reference number NCIB 12572.

2. An insecticidal formulation for combatting Coleoptera species which comprises as active ingredient an insecticidal δ-endotoxin produced by the stain A30.

3. An insecticidal formulation as claimed in claim 2 which further comprises a solid diluent or a surface active agent.

4. A process of protecting plants against attack by insects of the order Coleoptera which comprises exposing such attacking insects to the δ-endotoxin produced by the stain claimed in claim 1.

5. A process as claimed in claim 4 which comprises treating the plants prior to or during such attack with insecticidally effective amounts of a formulation claimed in either of claims 2 or 3.

6. A process as claimed in claim 4 in which the δ-endotoxin is produced within the plant by a gene derived from the strain claimed in claim 1.

7. A process as claimed in any of claims 4 to 6 in which the plant is potato or cotton.

8. A process as claimed in claim 7 in which the insect is Colorado potato beetle or cotton bollweevil.

9. A process as claimed in any of claims 4 to 6 in which the plant is maize (corn).

10. A process as claimed in claim 9 in which the insect is Southern or Western Corn Rootworm.

**Patentansprüche**

1. Der neue Stamm A30 des Bacillus thuringiensis, der bei den National Collections of Industrial and Marine Bacteria Scotland unter der Bezugs-nummer NCIB 12572 niedergelegt ist.

2. Insektizide Formulierung zur Bekämpfung von Coleoptera-Arten, welche als aktiven Bestandteil ein insektizides δ-Endotoxin enthält, das durch den Stamm A30 gebildet worden ist.

3. Insektizide Formulierung nach Anspruch 2, welche weiterhin ein festes Verdünnungsmittel oder ein oberflächenaktives Mittel enthält.

4. Verfahren zum Schützen von Pflanzen gegen den Angriff durch Insekten der Gattung Coleoptera, bei welchem die angreifenden Insekten dem δ-Endotoxin ausgesetzt werden, das durch den Stamm A30 gebildet wird.

**5.** Verfahren nach Anspruch 4, bei welchem die Pflanzen vor oder während des Angriffs mit einer insektizid wirksamen Menge einer Formulierung nach Anspruch 2 oder 3 behandelt werden.

**6.** Verfahren nach Anspruch 4, bei welchem das $\delta$-Endotoxin innerhalb der Pflanze durch ein Gen erzeugt wird, das sich von dem in Anspruch 1 genannten Stamm ableitet.

**7.** Verfahren nach einem der Ansprüche 4 bis 6, bei welchem die Pflanze eine Kartoffel- oder Baumwollpflanze ist.

**8.** Verfahren nach Anspruch 7, bei welchem das Insekt aus Colorado-Kartoffelkäfer oder Baumwollkapselkäfer besteht.

**9.** Verfahren nach einem der Ansprüche 4 bis 6, bei welchem die Pflanze aus Mais besteht.

**10.** Verfahren nach Anspruch 9, bei welchem das Insekt aus südlichem oder westlichem Maiswurzelwurm besteht.

**Revendications**

**1.** Souche nouvelle A30 de Bacillus thuringiensis, déposée dans les National Collections of Industrial and Marine Bacteria Scotland sous le numéro de référence NCIB 12572.

**2.** Formulation insecticide destinée à combattre des espèces de coléoptères, qui comprend comme ingrédient actif une $\delta$-endotoxine insecticide produite par la souche A30.

**3.** Formulation insecticide suivant la revendication 2, qui comprend en outre un diluant solide ou un agent tensio-actif.

**4.** Procédé de protection de plantes contre une attaque par des insectes de l'ordre des coléoptères, qui consiste à mettre en contact ces insectes parasites avec la $\delta$-endotoxine produite par la souche suivant la revendication 1.

**5.** Procédé suivant la revendication 4, qui consiste à traiter les plantes avant ou pendant une telle attaque avec des quantités à effet insecticide d'une formulation suivant la revendication 2 ou 3.

**6.** Procédé suivant la revendication 4, dans lequel la $\delta$-endotoxine est produite dans la plante par un gène dérivé de la souche suivant la revendication 1.

**7.** Procédé suivant l'une quelconque des revendications 4 à 6, dans lequel la plante est la pomme de terre ou le cotonnier.

**8.** Procédé suivant la revendication 7, dans lequel l'insecte est le doryphore ou le charançon des capsules de cotonnier.

**9.** Procédé suivant l'une quelconque des revendications 4 à 6, dans lequel la plante est le maïs.

**10.** Procédé suivant la revendication 9, dans lequel l'insecte est le ver des racines de maïs du Sud des Etats-Unis d'Amérique ou le ver des racines de maïs de l'Ouest des Etats-Unis d'Amérique.